Europäisches Patentamt

European Patent Office

Office européen des brevets

(19)

(11) Publication number: **0 199 074 B1**

## EUROPEAN PATENT SPECIFICATION

(12)

(45) Date of publication of patent specification: **20.03.91**

(51) Int. Cl.⁵ **A61B 17/11**

(21) Application number: **86103579.8**

(22) Date of filing: **17.03.86**

(54) Process for manufacturing an annealed prosthetic device.

(30) Priority: **25.03.85 US 715548**
**25.03.85 US 715547**

(43) Date of publication of application:
**29.10.86 Bulletin 86/44**

(45) Publication of the grant of the patent:
**20.03.91 Bulletin 91/12**

(84) Designated Contracting States:
**BE CH DE FR GB IT LI NL SE**

(56) References cited:
EP-A- 0 050 215    EP-A- 0 119 848
FR-A- 2 285 978    US-A- 3 516 957
US-A- 3 620 218    US-A- 4 404 161

R.M. OGORKIEWICZ (editor)
"THERMOPLASTICS: EFFECTS OF PROCESS-
ING"; 1969, THE CHEMICAL RUBBER CO.,
Cleveland, Ohio; pages 127-135

ENCYCLOPAEDIA OF POLYMER SCIENCE
AND TECHNOLOGY; vol. 8,1968; JOHN WILEY
& SONS, INC., page 63; vol. 4 1966, page 462;
vol. 9, 1968, page 145

LEO MANDELKERN "CRYSTALLIZATION OF
POLYMERS", 1964, McGRAW-HILL; pages
215-224

POLYMER, 1979, vol. 20, pages 1459-1464;
D.K. GILDING et al. "Biodegradable polymers
for use in surgery- polyglycolic/poly(actic
acid) homo- and copolymers: 1"

(73) Proprietor: **AMERICAN CYANAMID COMPANY**
**1937 West Main Street P.O. Box 60**
**Stamford Connecticut 06904-0060(US)**

(72) Inventor: **Mukherjee, Debi P.**
**73 Stony Hill Road**
**Brookfield Connecticut 06810(US)**
Inventor: **Feichtinger, Kurt A.**
**21 Tice Place**
**Ringwood New Jersey 07456(US)**

(74) Representative: **Wächtershäuser, Günter, Dr.**
**Tal 29**
**W-8000 München 2(DE)**

## Description

The present invention relates to a process for manufacturing an annealed prosthetic device from a synthetic bioabsorbable polymer in accordance with the preamble of Claim 1.

Such a process is known from EP-A-0 050 215.

An improved process for manufacturing an annealed prosthetic device from a synthetic bioabsorbable polymer has been invented. The improvement comprises holding the polymer in the mold at a temperature from above 70°C to 90°C. The posttreatment comprises annealing. In a preferred embodiment, the posttreatment comprises annealing and then sterilization. The polymer contains at least one glycolic acid ester linkage.

The device may have an ultimate flexural strain of up to about 6% and an ultimate flexural strength of about 22060N/cm² (32,000 psi). The device has a dimensional change during the annealing step of less than 0.48%.

In a preferred embodiment, the device is molded in an injection or blow or compression molding machine. In a specific embodiment, the compression molding machine is a transfer molding machine. In yet another preferred embodiment the mold comprises a casting.

In a further embodiment, the polymer is a homopolymer. In another further embodiment, the polymer is a copolymer. An improved anastomosis ring device may be molded according to the process of the invention.

## SUMMARY AND DESCRIPTION OF THE PROCESS

An improved process for manufacturing a molded prosthetic device from a synthetic bioabsorbable polymer has been invented which is characterised in Claim 1. The process comprises placing a solid amount of the polymer in a molding machine; melting the polymer by subjecting it to a temperature of from 240 to 255°C for about 2 to 5 minutes; transporting the polymer melt to an orifice; injecting the polymer melt from the orifice into a closed mold cavity at a pressure of from about 8000 to 9000 lbs per square inch (562 to 633 kg. per sq. cm.); holding the polymer in the mold cavity for less than one minute at a temperature from above 70 to 90°C; removing a molded prosthetic device from the mold cavity; and posttreating the molded prosthetic device. The posttreating step comprises annealing and then preferably sterilizing.

The polymer contains a glycolic acid ester linkage. In a more specific embodiment, the polymer is a homopolymer. In another more specific embodiment, the polymer is a copolymer.

Another embodiment with the posttreating step is wherein the molding mahcine is an injection or blow or compression molding machine. In a more specific embodiment, the compression machine is a transfer molding machine.

Yet another embodiment with the posttreating step is wherein the melting of the solid polymer is at a temperature of about 250°C. Still another embodiment is wherein the orifice has a temperature of about 240°C. Further another embodiment is wherein the injecting of the polymer melt is at a pressure of about 9000 lbs per square inch (633 kg/sq. cm). Finally, other embodiments are wherein the maximum thickness of the polymer in the mold cavity is about 6.35 mm (0.25 inches); wherein the holding of the polymer in the mold cavity is from about 20 to 30 seconds; and wherein the holding of the polymer is at a temperature of 90°C.

With the posttreating step comprising sterilizing, one embodiment is wherein the polymer is a homopolymer. Another embodiment is wherein the orifice has a temperature of about 240°C. A further embodiment is wherein the maximum thickness of the polymer in the mold cavity is about 6.35 mm (0.25 inch).

In another embodiment, the annealing is at a temperture of at least about 110°C under a vacuum of at least about 1004 mbar (755 mm of Hg). In a more specific embodiment, the annealing is at about 110°C under a vacuum of at least about 1006 mbar (757 mm of Hg). for about 2 to 3 hours.

I. Higher Mold Temperature To Reduce Dimen sional Changes Of Molded Devices Encountered During Annealing

Standard ASTM 6.35 cm x 1.27 cm x 0.32 cm (2-1/2" X 1/2" X1/8") flexural test samples were injection molded from PGA on an Arburg molding machine using various mold temperatures from 30.56°C (87°F) up to 90.0° C (194° F). Green strength and flexural (3 point bending) properties were measured, that is parts immediately removed from the machine. Flexural (3 point bending) properties as well as mold shrinkage were measured on "as-molded" parts after room temperature cooling. Shrinkage during annealing was determined from linear dimensional measurements taken both before and after annealing. Flexural (3 point bending) properties were also determined on the annealed samples. Specific details on the test procedures and results may be found in the tables.

The following terms are used in Tables 1 to 6:

A. Green Properties: Modulus and strength properties increased with mold temperature up to approximately 48.9° C (120° F) mold temperature, then stabilized at a lower value for mold temperatures from 60.0° C (140° F) to 90.0° C (194° F).

B. Linear Mold Shrinkage: Shrinkage dropped with increasing mold temperatures to a minimum at mold temperature of approximately 48.89° C (120° F) then increased slightly as the mold temperature was increased to 90.0° C (194° F).

C. "As molded" Flexural Properties: Modulus and strength properties increased by about 10% for mold temperatures above 82.22° C (180° F). Ultimate strain, which is a measure of ductility, uniformly increased with mold temperature. Thus, samples using a mold temperature above 82.22° C (180° F) were tougher, stronger and stiffer than those molded at 37.78° C (100° F) which is currently used to mold BAR devices.

D. Shrinkage During Annealing: The linear shrinkage which occurred during annealing for samples using mold temperatures above approximately 71.11° C (160° F) was very low and less than the experimetal error associated with the measurement.

E. Annealed Flexural Properites: The flexural (3 point bending) properties on annealed samples did not change with mold temperature.

Recommendations:

A. "Hot" molds (above 82.22° C (180° F)) should be employed when molding PGA where dimensional stability during annealing is desired.

B. Temperature control is crucial to part yield. An adequate controller is therefore required which can achieve this level of control.

Results And Discussion

The green properties of ultimate strength and flexural modulus increased with mold temperature, reaching a peak value at a mold temperature of 46.67° C (116° F) after which they dropped somewhat. Ultimate strain increased uniformly with increasing mold temperature. The linear shrinkage reached a minimum value at a mold temperature of approximately 46.67° C (116° F). Since the Tg of PGA is around 40.0° C (104° F) these observations are somewhat related to glass transition behaviour.

The flexural and ultimate properties of "as-molded" parts did not show any change until the mold temperature was increased above approximately 82.78° C (181° F). When the higher mold temperature samples were annealed, these properties did not change appreciably. However, the shrinkage during annealing of parts molded with mold temperatures higher than 70.0° C (158° F) was very low and within the range of the experimental error. These results are caused by a complex interaction of rates of cooling and crystallization at temperatures higher and lower than the glass temperature which is around 40.0° C (104° F) for PGA. Additional differential scanning colorimeter work may give more insight into the mechanism of these changes.

However, these changes indicate that higher mold temperatures will yield devices with a negligible degree of shrinkage during annealing. Therefore, higher mold temperatures are recommended to reduce shrinkage and possibly warpage of devices during annealing.

The results of part I are shown in the following Tables 1 to 6.

3

## Table 1 - Process Details

A. Materials: PGA, 1.34 I.V.

B. Equipment:
(1) 40 ton Clamp Arburg Model #220 - 90 - 350 (Serial # 118072) with 25 mm screw, nylon-type nozzle with negative taper, 1.55 oz. shot size.

(2) ASTM Mold #5129 with 2-1/2" X 1/2" X 1/8" flexural sample, end-gated.

C. Processing Parameters

Temperatures:

| | | |
|---|---|---|
| Feed Throat: | city water chilled | |
| Zone 1: | 490°F | (255°C) |
| Zone 2: | 475°F | |
| Zone 3: | 475°F | |
| Zone 4: (Nozzle) | 470°F | (243°C) |

Pressures*/Times:

| | |
|---|---|
| Initial Inject: | 8200 psi/3.5 sec. |
| Holding: | 3280 psi/1.4 sec. |
| Back: | None |
| Mold Cure: | 20.0 sec. |
| Overall Cycle: | 34 sec. |

Screw Speed: 50-70 rpm

*Specific Pressure

D. Samples: approximately 25 samples each at 87, 102, 116, 140, 158, 181 and 194°F mold temperatures (measured) were collected. Five each were measured immediately out of the mold for flexural 3-point bending properties. Five each were annealed at 110°C under vacuum for 3 hrs. The results are shown in Tables I to V below.

EP 0 199 074 B1

## TABLE 2 - PGA GREEN PROPERTIES IN FLEXURAL 3-POINT BENDING

Green Properties: samples immediately removed from the machine were degated and tested in flexure for three-point bending performance. A 2" span was used with 5 in/min crosshead and 50 in/min chart speeds to minimize cooling effects. A 20 lb. full scale range was used. Calculations for modulus, ultimate strength and strain follow ASTM #D-790 procedures. The results are as shown below.

### MOLD TEMPERATURE*

|  | 87°F | 102°F | 116°F | 140°F | 158°F | 181°F | 194°F |
|---|---|---|---|---|---|---|---|
| Flexural Modulus, (psi) | 119,000 | 130,000 | 159,000 | 118,000 | 104,000 | 112,000 | 106,000 |
|  | ±16,000 | ±9,330 | ±10,100 | ±9,050 | ±5,140 | ±10,000 | ±4,810 |
| Ultimate Strength, (psi) | 6,190 | 6,520 | 7,900 | 6,550 | 6,320 | 6,260 | 6,230 |
|  | ±590 | ±419 | ±323 | ±168 | ±327 | ±230 | ±111 |
| Ultimate Strain, (%)** | 8.64 | 8.99 | 9.22 | 9.04 | 9.04 | 9.24 | 9.32 |
|  | ±0.20 | ±0.23 | ±0.06 | ±0.30 | ±0.51 | ±0.08 | ±0.32 |

NOTES: Samples removed immediately from machine, degated and tested.
2" span
5 in/min. crosshead speed
50 in/min. chart speed
20 lb. full scale range
ASTM #D-790 procedure

\* Measured

\*\*Calculated strains much greater than 5-6% are not accurate due to inability to locate neutral axis.

EP 0 199 074 B1

## TABLE 3 - PGA LINEAR MOLD SHRINKAGE

Linear Mold Shrinkage:  Using the 2-1/2" length, average of each side of the gate, the ASTM #D-955 procedure was used to calculate mold shrinkage.

$$\text{Mold-Shrinkage (in/in)} = \frac{\text{Mold Cavity Dimension} - \text{Specimen Dimension}}{\text{Mold Cavity Dimension}}$$

### MOLD TEMPERATURE*

| | 87°F | 102°F | 116°F | 140°F | 158°F | 181°F | 194°F |
|---|---|---|---|---|---|---|---|
| Linear Mold Shrinkage, (in/in) | 0.0234 ±0.0005 | 0.0210 ±0.0013 | 0.0186 ±0.0015 | 0.0205 ±0.0009 | 0.0209 ±0.010 | 0.0226 ±0.0010 | 0.0214 ±0.0004 |

NOTES:  Shrinkage in 2-1/2" length dimension, average of lengths on right and left side of gate
ASTM #D-955 procedure

$$\text{Linear Mold Shrinkage (in/in)} = \frac{\text{Mold Cavity Dimension} - \text{Specimen Dimension}}{\text{Mold Cavity Dimension}}$$

*Measured

EP 0 199 074 B1

## TABLE 4 - PGA "AS MOLDED" PROPERTIES IN FLEXURAL 3-POINT BENDING

"As-Molded" Flexural Properties: samples which had cooled overnight in the dry locker were tested in flexure for three-point bending performance. A 2" span was used with 0.2 in/min crosshead and 5.0 in/min chart speeds. A 100 lb. full scale range was used. Calculations for modulus, ultimate strength and strain followed ASTM #D-790 procedures.

### MOLD TEMPERATURE*

|  | 87°F | 102°F | 116°F | 140°F | 158°F | 181°F | 194°F |
|---|---|---|---|---|---|---|---|
| Flexural Modulus, (psi) | $1.03 \times 10^6$ ±21,500 | $1.02 \times 10^6$ ±21,700 | $1.04 \times 10^6$ ±14,800 | $1.05 \times 10^6$ ±16,300 | $1.08 \times 10^6$ ±27,700 | $1.14 \times 10^6$ ±17,200 | $1.14 \times 10^6$ ±27,900 |
| Ultimate Strength, (psi) | 30,200 ±372 | 30,800 ±2,330 | 29,700 ±853 | 30,700 ±258 | 31,800 ±521 | 33,100 ±260 | 32,800 ±253 |
| Ultimate Strain, (%) | 4.40 ±0.12 | 4.56 ±0.12 | 4.62 ±0.25 | 5.17 ±0.08 | 5.13 ±0.13 | 5.24 ±0.10 | 5.35 ±0.12 |

NOTES: Molded samples allowed to cool overnight in dry locker prior to testing.
    2" span
    0.2 in/min. crosshead speed
    5.0 in/min. chart speed
    100 lb. full scale range
    ASTM #D-790 procedure

*Measured

EP 0 199 074 B1

## TABLE 5 - PGA LINEAR SHRINKAGE DURING ANNEALING

Shrinkage During Annealing: Similar to #2 above except the 2-1/2" length dimensions were measured before and after the 3 hrs. at 110°C vacuum-annealing schedule. Calculations were made using the following equation:

Shrinkage During Annealing (in/in) =

$$\frac{\text{Length After Annealing} - \text{Length Before Annealing}}{\text{Length before annealing}}$$

### MOLD TEMPERATURE*

| | 87°F | 102°F | 116°F | 140°F | 158°F | 181°F | 194°F |
|---|---|---|---|---|---|---|---|
| Linear Shrinkage During Annealing, (in/in) | $2.047 \times 10^{-3}$ $\pm 3.84 \times 10^{-4}$ | $2.044 \times 10^{-3}$ $\pm 3.87 \times 10^{-4}$ | $1.591 \times 10^{-3}$ $\pm 8.90 \times 10^{-4}$ | $1.998 \times 10^{-3}$ $\pm 3.01 \times 10^{-4}$ | $0.409 \times 10^{-3}$ $\pm 6.09 \times 10^{-4}$ | $0.451 \times 10^{-3}$ $\pm 4.08 \times 10^{-4}$ | $0.246 \times 10^{-3}$ $\pm 4.81 \times 10^{-4}$ |

NOTES: Shrinkage in 2-1/2" length dimension average of lengths on right and left side of gate, before and after 3 hours at 110°C/vacuum annealing treatment.

Linear shrinkage during annealing (in/in) = $\dfrac{\text{Length After Annealing} - \text{Length Before Annealing}}{\text{Length Before Annealing}}$

*Measured

EP 0 199 074 B1

## TABLE 6 - PGA "ANNEALED" PROPERTIES IN FLEXURAL 3-POINT BENDING

"Annealed" Flexural Properties:  identical to #3 above except samples were annealed under vacuum for 3 hours at 110°C and allowed to cool overnight in the dry locker prior to testing.

## TABLE 6 - PGA "ANNEALED" PROPERTIES IN FLEXURAL 3-POINT BENDING

### MOLD TEMPERATURE*

|  | 87°F | 102°F | 116°F | 140°F | 158°F | 181°F | 194°F |
|---|---|---|---|---|---|---|---|
| Flexural Modulus, (psi) | $1.07 \times 10^6$ ±43,700 | $1.04 \times 10^6$ ±29,500 | $1.06 \times 10^6$ ±23,600 | $1.10 \times 10^6$ ±32,200 | $1.11 \times 10^6$ ±29,600 | $1.13 \times 10^6$ ±11,700 | $1.10 \times 10^6$ ±6,950 |
| Ultimate Strength, (psi) | 31,300 ±755 | 30,900 ±538 | 31,700 ±471 | 32,300 ±446 | 32,500 ±665 | 33,000 ±209 | 32,300 ±318 |
| Ultimate Strain, (%) | 5.50 ±0.19 | 5.70 ±0.07 | 5.60 ±0.18 | 5.55 ±0.04 | 5.73 ±0.24 | 5.66 ±0.12 | 5.79 ±0.16 |

NOTES:  Molded samples were annealed under vacuum for 3 hrs. at 110°C then allowed to cool overnight in the dry locker prior to testing.
2" span
0.2 in/min crosshead speed
5.0 in/min chart speed
100 lb. full scale load
ASTM #D-790 procedure

*Measured

EP 0 199 074 B1

## II. Characterization of Injection-Molded Polyglycolic Acid Ligating Clips

Injection-molded polyglycolic acid (PGA) ligating clips were characterized for dimensional stability. The results indicate much improved dimensional stability utilizing a higher mold temperature.

An unacceptable breakage rate upon closure (approximately 30%) persisted despite the mold modifications to eliminate interference. In reducing the overall interference, sacrifices were made in initial clip clamping force to an unacceptable level of about 22.24N (5 lbs).

Recommendations to improve the breakage rate and the clamping force are to structurally change the clip design and to utilize a higher mold temperature to minimize dimensional changes during subsequent annealing. A higher mold temperature also allows the clips to be annealed with minimal fixturing.

### A. Process Description

Approximately 300 samples of a ligating clip were injection molded from PGA using the conditions listed in Table 7. While sinking was not apparent, flash problems persisted. The flash problem can be minimized by using a hydraulic molding machine with the proper control on the injection profile.

The samples were zylene washed and overnight vacuum dried at room temperature. They were then annealed free-standing for 3 hours at 110°C under vacuum, degated, deflashed and packaged for sterilization.

### B. Dimensional Change Characterization

Prior to annealing, the overall length of twenty clips was measured and each clip was uniquely identified. The lengths were taken again after annealing.

The dimensional changes in overall length for the above procedure are shown in Tables 8 to 10 with the higher mold temperature to limit the dimensional changes during annealing, an overall consistent shrinkage of approximately 0.003048 cm (0.0012 inch) from the original 0.9373 cm (0.369 inch) length was observed during the annealing process. This result may be contrasted to a 0.0254 cm (0.010 inch) shrinkage on cold molded samples. An approximately 90% improvement in shrinkage is obtained by utilizing the higher mold temperature.

Radiation sterilization of 2.5 Mrads created no detectable dimensional changes while ETO sterilization resulted in an average overall shrinkage of 0.001778 cm (0.0007 inch), but not with consistency. Within the standard deviation of the data, the shrinkage during ETO sterilization was insignificant.

The in -vitro results of part II are shown in the Tables 11 and 12.

## Table 7.

## Process Conditions Utilized For Injection-Molded Characterization

MATERIAL:  PGA

MACHINE :  Dynacast Mark MK-2 Serial #168-81
Plunger Type Pneumatic Injection -
Molding Machine

TEMPERATURES:  Heating Chamber: 470°F
Hot Runner Block: 475°F
Mold: 225°F/225°F: Front/Back

TIMES:  Initial Injection: 2 sec.
Overall Injection: 5 sec.
Overall Cycle:  40 sec.

Injection Pressure: 50 psi (max)

Injection Speed: 2 sec. (slow)

## Table 8

### Dimensional Length Changes Of Clips On Annealing

| Lot No. | Overall Length (inches) | | | Difference in Length$(L_O-L_1)$ | Average Difference $(L_O-L_1)$ |
|---|---|---|---|---|---|
| | Prior to Annealing $L_O$ | After Annealing $L_1$ | % Change | | |
| A. Average of 10 Samples | 0.3697 | 0.3686 | -0.2970% | .0011 | |
| Deviation | ±0.0015 | ±0.0011 | ±0.1652 | | .0012 |
| B. Average of 10 Samples | 0.3689 | 0.3676 | -0.3522% | .0013 | |
| Deviation | ±0.0010 | ±0.0008 | ±0.1303 | | |

EP 0 199 074 B1

## Table 9
### Dimensional Length Changes of Clips on Sterilization

#### Overall Length (inches)

| Lot No. | | After Annealing and prior to Sterilization[+] | Sterilization Used | After Sterilization | % Change |
|---|---|---|---|---|---|
| A. | Average of 10 Samples | 0.3686 | ETO | 0.3679 (9 samples) | -0.1507%* |
| | Deviation | ±0.0011 | | ±0.0011 | ±0.1430 |
| B. | Average of 10 Samples | 0.3676 | 2.5 Mrad | 0.3676 | 0.0000% |
| | Deviation | ±0.0008 | | ±0.0008 | |

+From Table 8

*7 samples

EP 0 199 074 B1

## Table 10

### Dimensional Length Changes Of Clips On Crimping

#### Overall Length (inches)

| Lot No. | After Annealing & Sterilization[+] | % Change | After Closure on Silastic® | % Change |
|---|---|---|---|---|
| A. Average of 9 Samples | 0.3679 | -0.1507%* | 0.3706* | 0.8168%* |
| Deviation | ±0.0011 | ±0.1430 | ±0.0005 | ±0.3157 |
| B. Average of 10 Samples | 0.3676 | 0.0000% | 0.3710* | 0.8942* |
| Deviation | ±0.0008 | | ±0.0008 | ±0.3772 |

+From Table 9

*7 samples

EP 0 199 074 B1

## TABLE 11

### In-Vitro Opening (Pull-apart) Force of Medium J-Clip

| Lot No. | No. of Samples | No. Of Days in Buffer | Pull-Apart Force (Average) | Failure Mode |
|---|---|---|---|---|
| A. | ETO Sterilized | | | |
| 1. | 7 | 0(Initial) | 5.34 ± 2.03 lb. | Teeth Shear Smooth |
| 2. | 5 | 1 Day | 3.05 ± 0.95 lb. | Teeth Shear Smooth |
| 3. | 4 | 3 Days | 3.72 ± 0.31 lb. | Teeth Shear Smooth |
| 4. | 4 | 5 Days | 3.01 ± 1.81 lb. | Teeth Shear Smooth |
| 5. | 4 | 7 Days | 0.45 ± 0.10 lb. | Crescent Ear Broke |
| 6. | 4 | 10 Days | All opened prior to testing | Crescent Ear Broke |
| B. | 2.5 Mrad Sterilized | | | |
| 1. | 7 | 0(Initial) | 5.04 ± 2.64 lb. | Teeth Shear Smooth |
| 2. | 5 | 1 Day | 2.11 ± 1.61 lb. | Teeth Shear Smooth |
| 3. | 5 | 3 Days | 1.55 ± 0.33 lb. | Teeth Shear Smooth |
| 4. | 4 | 5 Days | 0.61 ± 0.32 lb. | Crescent Ear Broke |
| 5. | 5 | 7 Days | 2 Samples opened prior to testing: 3 Samples crumbled in hands | |
| 6. | 5 | 10 Days | All opened prior to testing, crumbly | |

NOTE: Clips were annealed and either ETO or 2.5 Mrad sterilized and applied to 0.058" X 0.077" SILASTIC® (Dow Corning Co., U.S.A.) tubing to the full engagement of the teeth, immersed in pH 6.09 buffer @ 39°C. Pull-apart force measured at the applicator studs.

## TABLE 12

### In-Vitro Dynamic Pulse (Leak) Test of Medium J-Clip

| Lot No. | Sample No. | No. of Days to Leakage | Failure Mode |
|---|---|---|---|
| A. | ETO Sterilized | | |
| | 2 & 3 | 9 days | Crescent Ear Broke |
| | 4 to 6 | 10 days | Crescent Ear Broke |
| B. | 2.5Mrad Cobalt-60 Sterilized | | |
| | 2. | 7 days | Crescent Ear Broke |
| | 3 to 6 | 8 days | Crescent Ear Broke |

NOTE:
Clips were annealed, either ETO or 2.5Mrad sterilized and applied to 0.058" x 0.077" SILASTIC® tubing to the full engagement of the teeth, immersed in 6.09 PH buffer at 39°C. Periodically a 6psi average pressure was applied to the tubing and tube ends observed for leakage.

EP 0 199 074 B1

## Claims

1. A process for manufacturing an annealed prosthetic device from a synthetic bioabsorbable polymer containing a glycolic acid ester linkage comprising placing a solid amount of the polymer in a molding machine; melting said polymer by subjecting it to a temperature of from 240°C to 255°C; transporting the polymer melt to an orifice; injecting said melt from said orifice into a closed mold cavity; holding said polymer in said mold cavity for less than one minute; removing the molded prosthetic device from said cavity; and annealing said device, characterized by holding said polymer in said mold cavity at a temperature from above 70°C to 90°C, whereby a dimensional change of said device during the annealing step is less than 0.48 %.

2. A process according to Claim 1, wherein the molding machine is an injection or blow or compression molding machine.

3. A process according to Claim 1, wherein the maximum thickness of the polymer in the injection molding machine cavity is about 6.35 mm (0.25 inches).

4. A process according to Claim 1, comprising annealing and then sterilizing said prosthetic device, the annealing being at a temperature of at least about 110°C under a vacuum of at least about 1004 mbar (755 mm Hg).

5. A process according to Claim 4, wherein the annealing is at about 110°C under a vacuum of at least about 1006 mbar (757 mm Hg) for about 2 to 3 hours.

## Revendications

1. Procédé de fabrication d'une prothèse moulée recuite, à partir d'un polymère synthétique biorésorbable contenant une liaison ester d'acide glycolique, consistant à mettre une certaine quantité du polymère solide dans une machine à mouler, à faire fondre ledit polymère à une température de 240 à 255°C, à acheminer le polymère fondu vers un orifice, à injecter ledit polymère fondu, à travers ledit orifice, dans une cavité de moule fermée, à maintenir ledit polymère dans ladite cavité de moule pendant moins d'une minute, à retirer de la cavité du moule la prothèse moulée, et à recuire ladite prothèse, procédé caractérisé en ce que l'on maintient ledit polymère dans ladite cavité de moule à une température de plus de 70°C à 90°C, la modification dimensionnelle de ladite prothèse, lors du recuit, étant inférieure à 0,48%.

2. Procédé selon la revendication 1, dans lequel la machine à mouler est une machine à mouler par injection ou par soufflage ou par compression.

3. Procédé selon la revendication 1, dans lequel l'épaisseur maximale du polymère dans la cavité de la machine à mouler par injection est d'environ 6,35 mm (0,25 in).

4. Procédé selon la revendication 1, comprenant un recuit suivi d'une stérilisation de la prothèse, le recuit étant effectué à une température d'au moins environ 110°C et sous un vide d'au moins environ 1004 mbar (755 mm Hg).

5. Procédé selon la revendication 4, dans lequel le recuit est effectué à une température d'au moins environ 110°C et sous un vide d'au moins environ 1006 mbar (757 mm Hg) pendant 2 à 3 heures environ.

## Ansprüche

1. Verfahren zur Herstellung einer wärmebehandelten prothetischen Vorrichtung aus einem synthetischen, bioabsorbierbaren Polymeren, das eine Glycolsäureesterverknüpfung enthält, umfassend die Plazierung einer Feststoffmenge des Polymeren in eine Formmaschine; Schmelzen des Polymeren, indem man es einer Temperatur von 240°C bis 255°C aussetzt; Transportieren der Polymerschmelze zu einer

EP 0 199 074 B1

Mündung; Einspritzen der Schmelze aus der Mündung in eine geschlossene Formhöhle; Halten des Polymeren in der Formhöhle für weniger als eine Minute; Entfernung der geformten prothetischen Vorrichtung aus der Höhle; und Wärmebehandlung der Vorrichtung, dadurch gekennzeichnet, daß das Halten des Polymeren in der Formhöhle bei einer Temperatur von über 70° C bis 90° C erfolgt, wobei eine Dimensionsänderung der Vorrichtung während der Wärmebehandlungsstufe geringer ist als 0,48 %.

2.  Verfahren gemäß Anspruch 1, wobei die Formmaschine eine Spritz- oder Blas- oder Kompressions-Formmaschine ist.

3.  Verfahren gemäß Anspruch 1, wobei die maximale Dicke des Polymeren in der Spritzformmaschinen-Höhle etwa 6,35 mm (0,25 inch) beträgt.

4.  Verfahren gemäß Anspruch 2, umfassend die Wärmebenandlung und anschließende Sterilisierung der prothetischen Vorrichtung, wobei die Wärmebehandlung bei einer Temperatur von mindestens etwa 110° C unter einem Vakuum von mindestens etwa 1004 mbar (755 mm Hg) erfolgt.

5.  Verfahren gemäß Anspruch 4, wobei die Wärmebehandlung bei etwa 110° C unter einem Vakuum von mindestens etwa 1006 mbar (757 mm Hg) während etwa 2 bis 3 Stunden erfolgt.

18